# EUROPEAN PATENT SPECIFICATION

(11) **EP 1 341 751 B1**
(45) Date of publication and mention of the grant of the patent: **21.03.2007**
(21) Application number: 01989923.6
(22) Date of filing: 03.12.2001
(51) Int. Cl.: C07C 255/19, C07C 53/00, C07C 51/02, C07C 253/30, C07C 67/36

(54) **PROCESS FOR MAKING 5-CYANOVALERIC ACID OR ADIPIC ACID**
VERFAHREN ZUR HERSTELLUNG VON 5-CYANOVALERIANSÄURE ODER ADIPINSÄURE
PROCEDE DE FABRICATION DE L'ACIDE 5-CYANOVALERIQUE OU L'ACIDE ADIPIQUE

(30) Priority: 11.12.2000 US 254632 P
(43) Date of publication of application: 10.09.2003
(73) Proprietor: INVISTA Technologies S.à.r.l., Wilmington, DE 19808 (US)
(72) Inventor: BUNEL, Emilio, E., Indianapolis, IN 46032 (US); CLARK, David, A., Landenberg, PA 19350 (US)
(74) Representative: Cockerton, Bruce Roger
(86) International application number: PCT/US2001/046482
(87) International publication number: WO 2002/048094

(56) References cited:
- EP-A- 0 578 591
- WO-A-95/18783
- WO-A-98/42717
- DATABASE CA [Online] CHEMICAL ABSTRACTS SERVICE, COLUMBUS, OHIO, US; KAKUTA, ISAO ET AL: "Preparation of carboxylic acids or their esters using palladium catalysts and N-heterocycles" retrieved from STN Database accession no. 129:216347 XP002198096 & JP 10 218830 A (UBE INDUSTRIES, LTD., JAPAN) 18 August 1998 (1998-08-18)

## Description

The present invention relates to a process for the conversion of (1) 3-pentenenitrile to 5-cyanovaleric acid or (2) 3-pentenoic acid to adipic acid.

### BACKGROUND OF THE INVENTION

Two processes are known in the art for the preparation of caprolactam from butadiene. The first process involves the following steps: (1) stepwise addition of two equivalents of hydrogen cyanide to butadiene to produce adiponitrile, (2) partial hydrogenation of adiponitrile to form 6-aminocapronitrile, (3) separation of 6-aminocapronitrile from fully hydrogenated hexamethylenediamine and unreacted adiponitrile, and (4) hydrolysis of 6-aminocapronitrile and cyclization of the hydrolysis product to produce caprolactam.

The second process involves the following steps: (1) carbonylation of butadiene to methyl 3-pentenoate, (2) hydroformylation of methyl 3-pentenoate to methyl 5-formylvalerate, (3) reductive amination of methyl 5-formylvalerate to methyl 6-aminocaproate, and (4) cyclization of methyl 6-aminocaproate to caprolactam.

It would be desirable to develop a process that produces fewer by-products than the above processes and involves fewer reaction steps. A process that satisfies these needs could be based on the carbonylation of 3-pentenenitrile to 5-cyanovaleric acid or its corresponding esters. The process to produce caprolactam would involve the steps of: (1) hydrocyanation of butadiene to 3-pentenenitrile, (2) carbonylation of 3-pentenenitrile to 5-cyanovaleric acid or ester, and (3) hydrogenation and cyclization of 5-cyanovaleric acid or ester to caprolactam.

In order to develop a successful process, an active, selective catalyst that operates under mild conditions for the carbonylation of 3-pentenenitrile is needed. Previous attempts to produce methyl 5-cyanovalerate or 5-cyanovaleric acid from 3-pentenenitrile were based on the use of a cobalt catalyst. US patent 5,434,290 discloses the use of a cobalt catalyst, an activating solvent comprising carbonic diesters, carbamates or ureas and CO pressures between 210 to 270 bar to convert 3-pentenenitrile to methyl 5-cyanovalerate. US patent 4,508,660 discloses a similar process, but using sulfones as the preferred solvent and CO pressures between 14 to 35 MPa. The rates of carbonylation reported in this patent are quite low, the turnover frequency calculated for example 2 of U.S. Patent 4,508,660 gives 1.52 mol/mol-hour. A similar situation is described in U.S. patent 4,933,483.

Palladium-based catalysts for the carbonylation of olefins and diolefins are known in the art. U.S. Patent 5,028,734 discloses a process for the selective carbonylation of conjugated dienes in the presence of an alcohol and a catalyst system comprising a halide-free palladium salt, a bidentate phosphine ligand and a protonic acid with a pKa value greater than 3. PCT patent application WO 00/56695 discloses a process for the carbonylation of conjugated dienes by reaction with CO and alcohol in the presence of a catalyst system including a source of palladium cations, a phosphorus-containing ligand of structure X¹-R-X² and a source of anions. The preferred ligands are based on a 9-phosphabicyclononyl group for X¹ and X² and a simple bridge for R. PCT patent application WO97/38964 describes the use of the same catalyst system for the carbonylation of ethylenically unsaturated compounds.

PCT patent application WO 98/42717 describes a catalyst of the formula R¹>P-R²-PR³R⁴ for the carbonylation of terminal and internal olefins. The R¹>P moiety is a substituted 2-phosphatricyclo[3.3.1.1{3,7}] decyl group where one or more of the carbon atoms are replaced by heteroatoms, in particular oxygen. Comparisons are made between phosphines such as (Me₃C)P(CH₂)₃P(CMe₃) (DTBPP) and 1,3-P,P'-di (2-phospha-1,3,5,7-tetramethyl-6,9,10-trioxatricyclo[3.3.11{3.7}]decyl propane (DPA3). Carbomethoxylation of an internal-C14 olefin feed with a palladium-based catalyst using DPA3 as a ligand gives linear methyl ester with 78% selectivity at an average rate of 120 mol/mol-hour. In contrast, using DTBPP as ligand under indentical conditions gives only an average rate of 5 mol/mol-hour.

PCT patent application WO96/19434 and *Chem Comm.,* 1999, 20, 1877-1878 describe the use of bidentate phosphines such as (di-t-butyl phosphino)-o-xylene for the carbonylation of ethylene to methylpropanoate. The patent application describes catalysts using these bidentate phosphines as being unable to carbonylate propene. One skilled in the art might reason that if such catalysts are able to carbonylate ethylene, but fail to carbonylate propene at any appreciable rate, then these same catalysts would be inactive for the carbonylation of internal olefins. Surprisingly, these catalysts are able to convert an internal olefin, namely 3-pentenenitrile, to the corresponding linear carboxylic acid, namely 5-cyanovaleric acid (or its alkyl esters). Additionally these catalysts are able to convert 3-pentenoic acid to adipic acid, and to convert methyl 3-pentenoate to dimethyl adipate.

JP-A-10218830 discloses a process for obtaining carboxylic esters or carboxylic acids by reacting an olefin with carbon monoxide, an alcohol or water in the presence of palladium, phosphine or arsine of bidentate coordination, a specific acid and an N-heterocyclic base.

### SUMMARY OF THE INVENTION

The present invention is a process for producing (1) a compound of the formula NC-CH₂-CH₂-CH₂-CH₂-CO2H, or (2) adipic acid, comprising contacting a substrate selected from the group consisting of (A) and (B), wherein substrate (A) is 3-pentenenitrile, and wherein substrate (B) is 3-pentenoic acid, with water as a nucleophile, and carbon monoxide, in the presence of a Group VIII metal; a ligand having a formula 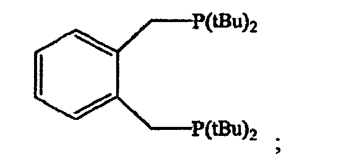 and a promoter, said promoter comprising a strong acid having a pKa in water of less than 1 and at least one carboxylic acid.

### DETAILED DESCRIPTION OF THE INVENTION

Disclosed herein is a process for preparing important, commercially useful compounds, each having various functionality. The catalyst, which is the metal plus the ligand, involved in the process of the present invention are very active and selective for the carbonylation of 3-pentenenitrile when compared with other prior art catalysts based on ligands such as 1,3-P,P'-di(2-phospha-1,3,5,7-tetramethyl-6,9,10-trioxatricyclo[3.3.11{3.7}]decyl propane and 1,2-P,P'bis(1,5-dimethyl, 9-phosphabicyclononyl)ethane.

Suitable Group VIII metals include cobalt, nickel, palladium, rhodium and platinum. Particularly preferred is palladium. The palladium catalyst used in the process of the invention may be provided in the form of a palladium complex of the specified bidentate phosphine. It may also be generated in situ by adding a source of palladium and a source of the bidentate phosphine to the reaction. Suitable sources of palladium include palladium carboxylates, such as palladium acetate, propionate, butyrate or benzoate and palladium salts of mineral acids. Further sources include palladium complexes such as palladium acetylacetonate, tris(dibenzylideneacetonate) dipalladium.

Some examples of suitable specific promoters include strong acids (pKa in water of less than 1), such as methanesulphonic acid, chlorosulphonic acid, benzene sulphonic acid, trifluoromethane sulphonic acid. When the nucleophile is water, and the substrate is 3-pentenenitrile, the strong acid must be combined with at least one carboxylic acid, such as acetic acid, propionic acid, benzoic acid, o-toluoic acid, m-toluoic acid, or p-toluoic acid.

The process may be carried out at a temperature in the range of 80 degrees C to 150 degrees C and a carbon monoxide partial pressure in the range of 200 to 2000 psi.

Suitable solvents are one or more aprotic solvents such as ethers, e.g. diethyl ether, diethylene glycol dimethyl ether, anisole; aromatic compounds, e.g. benzene, toluene, o-xylene, m-xylene, p-xylene; alkanes, e.g. hexane, heptane; nitriles, e.g. acetonitrile, benzonitrile, adiponitrile; and esters, e.g. methyl benzoate, ethyl acetate. The reaction also can be carried out using the substrates and alcohol and/or water as the solvent.

The molar ratio of the substrate and the water can vary widely in the range of 1:1 to 1:50, preferably between 1:1 to 1:10. The molar ratio of the substrate to the Group VIII metal can vary widely in the range 10:1 to 10000:1, preferably in the range of 100:1 to 2000:1. The molar ratio of the ligand to the Group VIII metal can vary widely in the of range 1:1 to 50:1, preferably in the range of 1:1 to 5:1. The molar ratio of the strong acid to the Group VIII metal can vary widely in the range 1:1 to 50:1, preferably in the range 1:1 to 5:1. The molar ratio of the carboxylic acid to the Group VIII metal can vary between widely in the range about 0:1 to about 10000:1, preferably in the range of about 1:1 to about 2000:1.

Bis(di-t-butyl phosphino)-o-xylene has been described in *J. Chem. Soc.*, *Chem. Comm,* 1976, 365, and it is made by treating o-BrCH₂C₆H₄CH₂Br with HP(t-Bu)₂ and subsequent reaction with a base.

The carbonylation process of the present invention can be performed batchwise, semi-continuously or continuously. Preferably a continuous manner of operation is used because it allows for higher molar ratios of substrate to the Group VIII metal and lower residence times. The products of the carbonylation of 3-pentenoic acid with water include adipic acid, 2-methylglutaric acid, and 2-ethylsuccinic acid.

The invention is illustrated by the following nonlimiting examples, in which the following terms are defined as indicated:
linearity: 100*[Moles of linear isomer]/[Sum of all isomers]
conversion: 100* [Substrate] / [Substrate] ₀ where [Substrate]₀ is the initial concentration of substrate selectivity: 100* [Moles of linear product] / [Sum of all products detected by GC analysis]
TOF (turnover frequency): [Moles of linear product] / [Moles of palladium] [hour]

### EXAMPLES

### Example 1: Synthesis of Ligand 1

Di-t-butyl phosphine (5.0 g, 0.0342 mol) was added dropwise to a solution of α,α'-dibromo-o-xylene (4.51 g, 0.0171 mol) in 50 ml acetone and left to stir for 36 hours at ambient temperature in a nitrogen drybox. After removing the acetone under vacuum, the resulting white solid was rinsed with ether. Under nitrogen, a solution of sodium acetate (12 g) in water (30 ml) was added to a suspension of the dry white solid in ether (150 ml). The product was extracted under nitrogen with ether (2 x 150 ml) and dried over NaSO₄. The combined ether layers were vacuum-stripped to yield 1.5 g of yellow solid (22% yield) . ³¹P NMR (C₆D₆) : 25. 1 ppm.

### Example 2: Hydrocarboxylation of 3-pentenitrile with Ligand 1

A 100 ml Hastelloy B air motor stirred Parr (brand name) reactor was loaded with a solution of the following composition: 34 g diglyme, 54 mg palladium acetate, 1.0 g 3-pentenenitrile, 46 mg methanesulfonic acid, 0.5.g o-dichlorobenzene, 0.143 g Ligand 1, 5 ml degassed water and 10g degassed acetic acid. After pressurizing the reactor to 500 psi of CO at the final temperature of 100 °C, the reaction was monitored by GC during the five hour run. Results for linearity, conversion, selectivity and turnover frequency (mol/mol h) are listed in Table 1.

**Table 1**

| **Time [min]** | **Linearity** | **Conversion** | **Selectivity** | **TOF** |
|---|---|---|---|---|
| 15.00 | 98.57 | 11.74 | 53.56 | 130.94 |
| 30.00 | 98.02 | 22.30 | 70.47 | 150.90 |
| 45.00 | 97.51 | 31.62 | 85.69 | 152.10 |
| 60.00 | 97.43 | 36.21 | 88.49 | 131.10 |
| 90.00 | 99.32 | 48.45 | 94.73 | 121.99 |
| 120.00 | 99.33 | 50.37 | 95.79 | 102.62 |
| 360.00 | 97.39 | 66.16 | 95.19 | 44.05 |

### Comparative Example B

1,3-P,P'-di(2-phospha-1,3,5,7-tetramethyl-6,9,10-trioxatricyclo[3.3.1.1{3.7}]propane (0.149 g), prepared as described in Example 1 of PCT patent application 98/42717, was substituted for Ligand 1 in the procedure outlined in Example 2. Results for linearity, conversion, selectivity and turnover frequency (mol/mol h) are listed in Table 2.

**Table 2**

| **Time [min]** | **Linearity** | **Conversion** | **Selectivity** | **TOF** |
|---|---|---|---|---|
| 15.00 | 86.08 | 1.45 | 71.96 | 89.04 |
| 30.00 | 86.34 | 4.75 | 77.06 | 68.09 |
| 45.00 | 86.64 | 5.48 | 78.72 | 57.18 |
| 60.00 | 86.91 | 8.29 | 80.37 | 55.32 |
| 90.00 | 87.00 | 11.25 | 81.66 | 50.96 |
| 120.00 | 87.06 | 16.72 | 82.72 | 48.69 |
| 180.00 | 87.23 | 22.54 | 84.08 | 43.81 |
| 240.00 | 87.16 | 28.02 | 84.51 | 37.77 |
| 300.00 | 87.14 | 29.18 | 84.79 | 31.52 |
| 360.00 | 87.10 | 27.87 | 85.00 | 26.51 |

## Claims

1. A process for producing
(1) a compound of the formula NC-CH₂-CH₂-CH₂-CH₂-CO₂H, or
(2) adipic acid,
comprising: contacting a substrate selected from the group consisting of (A) and (B) wherein substrate (A) is 3-pentenenitrile, and
wherein substrate (B) is 3-pentenoic acid,
with water as a nucleophile,
and carbon monoxide, in the presence of a Group VIII metal;
a ligand having a formula 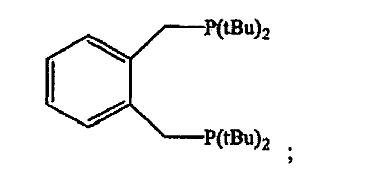 and a promoter, said promoter comprising a strong acid having a pKa in water of less than 1 and at least one carboxylic acid.

2. The process of claim 1 wherein said Group VIII metal is palladium.

3. The process of claim 1 wherein said promoter is methanesulphonic acid.

4. The process of claim 1 wherein the substrate is 3-pentenenitrile.

5. The process of claim 1 wherein the carboxylic acid is acetic acid.

## Patentansprüche

1. Verfahren zur Herstellung
(1) einer Verbindung der Formel NC-CH₂-CH₂-CH₂-CH₂-CO₂H oder
(2) einer Adipinsäure,
das die folgenden Schritte beinhaltet: Inkontaktbringen eines Substrats, ausgewählt aus der Gruppe bestehend aus (A) und (B),
wobei das Substrat (A) 3-Pentennitril ist und
wobei das Substrat (B) 3-Pentensäure ist,
mit Wasser als Nucleophil
und Kohlenmonoxid in Anwesenheit eines Metalls der Gruppe VIII,
einem Ligand mit der Formel 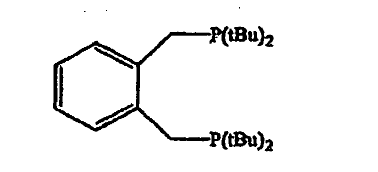 und einem Promotor, wobei der Promotor eine starke Säure mit einem pKa in Wasser von weniger als 1 und wenigstens eine Carbonsäure umfasst.

2. Verfahren nach Anspruch 1, wobei das Metall der Gruppe VIII Palladium ist.

3. Verfahren nach Anspruch 1, wobei der Promotor Methansulfonsäure ist.

4. Verfahren nach Anspruch 1, wobei das Substrat 3-Pentennitril ist.

5. Verfahren nach Anspruch 1, wobei die Carbonsäure Essigsäure ist.

## Revendications

1. Procédé de production
(1) d'un composé de formule NC-CH₂-CH₂-CH₂-CH₂-CO₂H, ou
(2) d'acide adipique,
comprenant: la mise en contact d'un substrat choisi parmi le groupe constitué de (A) et (B)
dans lequel le substrat (A) est le 3-pentènenitrile, et
dans lequel le substrat (B) est l'acide 3-penténoïque,
avec de l'eau comme substance nucléophile,
et du monoxyde de carbone, en présence d'un métal du groupe VIII ;
un ligand de formule 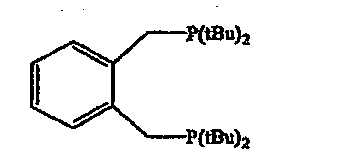 et un promoteur, ledit promoteur comprenant un acide fort ayant un pKa dans l'eau inférieur à 1 et au moins un acide carboxylique.

2. Procédé selon la revendication 1, dans lequel ledit métal du groupe VIII est le palladium.

3. Procédé selon la revendication 1, dans lequel ledit promoteur est l'acide méthanesulfonique.

4. Procédé selon la revendication 1, dans lequel le substrat est le 3-pentènenitrile.

5. Procédé selon la revendication 1, dans lequel l'acide carboxylique est l'acide acétique.
